# EUROPÄISCHE PATENTANMELDUNG

(11) **EP 2 737 809 A1**
(43) Veröffentlichungstag der Anmeldung: **04.06.2014**
(21) Anmeldenummer: 12195226.1
(22) Anmeldetag: 03.12.2012
(51) Int. Cl.: A23L 1/304, A23L 1/305, A23L 2/52, A61K 31/405, A61K 31/4415, A61K 31/198

(54) **Aminosäurehaltiges Getränk, geeignet zur Verwendung in der Prophylaxe und Behandlung von psychischen Störungen**

(71) Anmelder: MüMed, 81241 München (DE)
(72) Erfinder: Lindermüller, Dr. Anton, 82237 Wörthsee (DE); Reitzle, Dr. Karl, 80639 München (DE)
(74) Vertreter: Pfenning, Meinig & Partner GbR

(57) **Zusammenfassung**

Die Erfindung betrifft ein vitamin- und aminosäurehaltiges Getränk, insbesondere ein Erfrischungsgetränk oder Energiegetränk, welches speziell auf die biologischen und neuropsychologischen Anforderungen von Kindern und Jugendlichen abgestimmt wurde. Das erfindungsgemäße Getränk eignet sich zur Prophylaxe und/oder Behandlung von psychischen Störungen, besonders zur Prophylaxe und/oder Behandlung von Aufmerksamkeitsdefizithyperaktivitätssyndrom (ADHS) und/oder Aufmerksamkeitsdefizitsyndrom (ADS).Das erfindungsgemäße Getränk enthält Phenylalanin, Tyrosin sowie Tryptophan als Aminosäuren, in einer ausgewählten Konzentration und Vitamin B6. Die Erfindungs betrifft weiterhin die Verwendung des Getränks zur Prophylaxe und/oder Therapie von ADHS oder ADS.

## Beschreibung

Die Erfindung betrifft ein vitamin- und aminosäurehaltiges Getränk, insbesondere ein Erfrischungsgetränk oder Energiegetränk, welches speziell auf die biologischen und neuropsychologischen Anforderungen von Kindern und Jugendlichen abgestimmt wurde. Das erfindungsgemäße Getränk eignet sich zur Prophylaxe und/oder Behandlung von psychischen Störungen, besonders zur Prophylaxe und/oder Behandlung von Aufmerksamkeitsdefizithyperaktivitätssyndrom (ADHS) und/oder Aufmerksamkeitsdefizitsyndrom (ADS).Das erfindungsgemäße Getränk enthält Phenylalanin, Tyrosin sowie Tryptophan als Aminosäuren, in einer ausgewählten Konzentration und Vitamin B6. Die Erfindungs betrifft weiterhin die Verwendung des Getränks zur Prophylaxe und/oder Therapie von ADHS oder ADS.

Es gibt eine Vielzahl von Erfrischungsgetränken. Dazu zählen z.B. Mineralwässer mit und ohne Kohlensäure oder Aromazusätzen, außerdem Limonaden und Fruchtsaftgetränke, welche manchmal mit Vitaminen versetzt werden. Diese Getränke dienen meist nur der Aufnahme der täglich benötigten Flüssigkeit. Alle wichtigen Nährstoffe werden hingegen meist mit der Nahrung aufgenommen.

Jedoch ist es in fast allen Industrieländern zu einer Entwicklung gekommen, aufgrund welcher eine ausgewogene Ernährung meist nicht mehr stattfindet. Dies ist für die meisten erwachsenen Menschen weniger von Bedeutung, da sie gewisse Mängel über eine bestimmte Zeit relativ gut ausgleichen und/oder auf verschiedene Nahrungsergänzungen zurückgreifen können, welche sie vor einem Mangel bewahren.

Kinder und Jugendliche hingegen befinden sich noch in einer Entwicklungsphase, in welcher eine ausgewogene Ernährung besonders wichtig ist. Des Weiteren können Kinder und Jugendliche einen Mangel nicht ohne das Risiko einer Entwicklungsschwäche verkraften. Hinsichtlich der Einnahme von Nahrungsergänzungen sind sie aber entweder sehr eingeschränkt oder diese ist gar nicht möglich, da solche Präparate eher für erwachsene Menschen ausgelegt sind.

Zum einen neigen Kinder und Jugendliche generell dazu, zu wenig zu trinken.

Zum anderen sind die bisherigen Getränke wie z.B. Mineralwässer mit und ohne Kohlensäure in Verbindung mit Aromazusätzen, sowie Limonaden und Fruchtsaftgetränke fast alle als ungesund für Kinder und Jugendliche einzustufen, da sie zumeist zu viel Zucker und Aromastoffe enthalten bzw. überhaupt solche Stoffe beinhalten. Auch hier soll die Erfindung Abhilfe schaffen.

Ein weiterer wichtiger Punkt ist der, dass Kinder und Jugendliche immer höheren Ansprüchen innerhalb ihrer Umwelt ausgesetzt sind, vor allem im Bereich des Lernens und der Schule. Außerdem kommen, wie eingangs schon erwähnt, oft noch eine vernachlässigte und ungesunde Ernährung hinzu, so dass keine ausreichende Nährstoffversorgung besteht. Für Kinder und Jugendliche kann somit ein Mangel bestehen der sich negativ für ihren Alltag auswirkt.

Erwachsene Menschen können hierbei auf Nahrungsergänzungen und insbesondere sogenannte "Energy Drinks" und Cola-Getränke zurückgreifen. Dies können Kinder und Jugendliche jedoch nicht ohne Gefahren, da diese Mittel und Getränke zumeist auf erwachsene Menschen ausgelegt sind und schädlich für Menschen sein können, welche sich noch in der Entwicklungsphase befinden.

Weitere Getränke, welche hier als Beispiele aufgeführt werden können, greifen auch wieder auf Koffein sowie auf Fettsäuren zurück (WO 2011/120969 A1 und DE 10 2007 030 495 A1).

Vor allem koffeinhaltige Getränke sind für Kinder und Jugendliche bedenklich, Bei Nahrungsmittelergänzer wie Omegafettsäuren sind deren Auswirkung auf die Leistungsfähigkeit des Gehirns noch nicht hinreichend belegt. Ebenso ist noch nicht ausgeschlossen, dass durch übermäßige Zufuhr von Omegafettsäuren ein Risiko für eine Störung des Fettstoffwechsels entstehen kann. Hierbei sind insbesondere Kinder und Jugendliche in ihrer Entwicklung betroffen,

Es ist zu erwähnen, dass es auch in diesem Bereich schon Produkte gibt, welche bereits Aminosäuren beinhalten. Jedoch sind die Zusammensetzungen in diesen Produkten entweder nicht auf Kinder und Jugendliche ausgelegt und/oder beinhalten wieder weitere Stoffe, welche im vorherigen Absatz genannt wurden und bedenklich für Kinder und Jugendliche sind. Als Beispiele sind hier Patent DE 10 2005 006 241 A1, sowie Patent US 6,261,589 B1 zu nennen, welches auf Aminosäuren zurückgreifen, aber auch wieder das für Kinder schädliche Koffein und Taurin enthalten. Des Weiteren Patent EP2 095 728 B1, welches das für Kinder schädliche Glutamin enthält.

Infolge der soeben dargelegten Argumente können Kinder und Jugendliche nicht auf die bisherigen Getränke, Lebensmittel und Nahrungsergänzungen zurückgreifen, ohne ihre Gesundheit und Entwicklung zu gefährden.

Die Aufgabe der vorliegenden Erfindung ist somit die Bereitstellung eines Nahrungsergänzungsmittels, welches besonders die neurobiologischen Voraussetzungen einer optimalen Hirnfunktion von Kindern unterstützt.

Das Problem wird gelöst durch das aminosäurehaltige Getränk gemäß Anspruch 1 und die Verwendung des aminosäurehaltigen Getränks gemäß Anspruch 12.

Erfindungsgemäß wird ein aminosäurehaltiges Getränk bereitgestellt, enthaltend auf 200 Gramm Wasser
- 440-880 mg Phenylalanin,
- 290-580 mg Tyrosin sowie
- 96-192 mg Tryptophan,
   mit der Maßgabe, dass die Menge von Phenylalanin größer als die Menge von Tyrosin und Tryptophan und die Menge von Tyrosin größer ist als die Menge an Tryptophan ist, sowie
- 0,5-1,0 mg Vitamin B6.

Der Vorteil des erfindungsgemäßen Getränks ist, dass es aus natürlichen und für Kinder ungefährlichen Stoffen besteht. Weiter wird durch den Konsum des Getränkes, die Leistungsfähigkeit gesteigert, indem die natürlichen Resourcen unterstützt werden, ohne das dies über die sogenannten "Energy Drinks" oder Getränke auf Basis von Koffein und/oder Taurin erfolgt. Es hat sich herausgestellt dass es beim erfindungsgemäßen Getränk besonders wichtig ist die in Anspruch 1 definierten Konzentrationen einzuhalten. Wesentlich ist dabei dass in allen Fällen die enthaltene Menge von Phenylalanin immer größer ist als die Menge von Tyrosin bzw. von Tryptophan. Zusätzlich muss die enthaltene Menge von Tyrosin immer größer sein als die Menge von Tryptophan. Es hat sich gezeigt dass die Einhaltung dieser Bemessungsregel für den Erfolg der Erfindung wesentlich ist.

In einer bevorzugten Ausführungsform kann das erfindungsgemäße Getränk zusätzlich als Mineralstoffe 5 mg - 8 mg Zink und/oder 120 mg - 240 mg Magnesium enthalten.

Ferner kann das Getränk zusätzlich als Aminosäuren Arginin und/oder Histidin enthalten.

Bevorzugt weist das Getränk zusätzlich als Vitamine die Vitamine Vitamin C, Vitamin D, Vitamin B1, Vitamin B2, Vitamin B7 und/oder Vitamin B12 auf.

In einer bevorzugten Ausgestaltungsform sind desweiteren als zusätzliche Mineralstoffe Eisen und/oder Calcium enthalten.

Besonders bevorzugt enthält das erfindungsgemäße Getränk
- 440-880 mg Phenylalanin,
- 290-580 mg Tyrosin,
- 96-192 mg Tryptophan,
- 500-1000 mg Arginin,
- 240-480 mg Histidin sowie
- 75-90 mg Vitamin C,
- 0,04-0,005 mg Vitamin D,
- 1,0-1,1 mg Vitamin B1,
- 1,0-1,3 mg Vitamin B2,
- 0,5-1,0 Vitamin B6,
- 0,013-0,025 mg Vitamin B7 sowie
- 1,5-2.0 mg Vitamin B12 und
- 8,0-14 mg Eisen,
- 5,0-8,0 mg Zink,
- 700-1100 mg Calcium und
- 120-240 mg Magnesium

In einer weiteren bevorzugten Ausgestaltungsform ist das Getränk aus Wasser und zugesetzten Fruchtsäften sowie zugesetzten Aminosäuren, Vitaminen und/oder Mineralstoffen gebildet. Diese Ausführungsform hat den Vorteil das ausgehend von einer natürlichen Quelle d.h. einem Saft der schon Teile der erforderlichen Komponenten enthält , die restlichen noch benötigten Komponenten nur noch zugesetzt werden müssen. Als besonders geeignet hat sich hierbei Holundersaft erwiesen. Holundersaft enthält bereits aufgrund der Fruchtbasis die wesentlichen Aminosäure, Vitamine und Mineralstoffe, sodass Diese Inhaltsstoffe noch hinzugefügt werden müssen um die georderten Konzentrationen zu erreichen.

Desweiteren kann das Getränk je nach Geschmack Kräuter-, Tee-, Gemüse-, Obst- und/oder Gewürzauszüge enthalten. Die Errfindung unterliegt hierbei keiner beschränkung.

Da in dem erfindungsgemäßen Getränk zu Erreichung der gewünschten Eigenschaften kein Taurin und/oder Koffein enthalten sein muss enthält das Getränk in einer besonders bevorzugten Ausgestaltungsform kein Koffein und/oder kein Taurin.

Eine bevorzugte Zusammensetzung des Getränks wird in der nachfolgenden Tabelle 1 näher beschrieben. Die angegebenen Mengen der Aminosäuren, Vitamine und Mineralstoffe beziehen sich auf ein Getränk von 200 ml. Das Getränk kann dabei entweder nur aus Wasser und zugesetzten Wirkstoffen bestehen oder das Getränk kann auf Basis eine Saftes wie zum Beispiel Holundersaft und zugesetzten Mengen an Inhaltsstoffen bestehen. In der Tabelle ist in der 1. Spalte eine Zusammensetzung angegeben die auf Basis Von Fruchtsaft und Wasser gebildet wird.

| **Tabelle 1** | | | | |
|---|---|---|---|---|
| **Flüssigkeit** | **Min.** | | **Max.** | **Mittelwert** |
| Mineralwasser | 80 ml | bis | 120 ml | 100 ml |
| Fruchtsaft | 80 ml | bis | 120 ml | 100 ml |
| | | | | |

| **Aminosäuren** | **Min.** | | **Max.** | **Mittelwert** |
|---|---|---|---|---|
| Phenylalanin | 440 mg | bis | 880 mg | 660 mg |
| Tyrosin | 290 mg | bis | 580 mg | 435 mg |
| Tryptophan | 96 mg | bis | 192 mg | 144mg |
| Arginin | 500 mg | bis | 1000 mg | 750 mg |
| Histidin | 240 mg | bis | 480 mg | 360 mg |
| | | | | |

| **Vitamine** | **Min.** | | **Max.** | **Mittelwert** |
|---|---|---|---|---|
| Vitamin C | 75 mg | bis | 90 mg | 82,5 mg |
| Vitamin D | 0,004 mg | bis | 0,005 mg | 0,0045 mg |
| Vitamin B1 | 1,0 mg | bis | 1,1mg | 1,05 mg |
| Vitamin B2 | 1,0 mg | bis | 1,3 mg | 1,15mg |
| Vitamin B6 | 0,5 mg | bis | 1,0 mg | 0,75 mg |
| Vitamin B7 | 0,013 mg | bis | 0,025 mg | 0,019 mg |
| Vitamin B12 | 1,5 mg | bis | 2,0 mg | 1,75 mg |
| | | | | |

| **Mineralstoffe** | **Min.** | | **Max.** | **Mittelwert** |
|---|---|---|---|---|
| Eisen | 8,0 mg | bis | 14 mg | 11 mg |
| Zink | 5,0 mg | bis | 8,0 mg | 6,5 mg |
| Calcium | 700 mg | bis | 1100 mg | 950 mg |
| Magnesium | 120 mg | bis | 240 mg | 180 mg |

Wie deutlich anhand der Zusammensetzung zu erkennen ist, enthält diese Ausgestaltungsform des Getränks mehrere Aminosäuren. Dies liegt zum einen daran, dass Aminosäuren Ausgangssubstanzen für die Synthese wichtiger Neurotransmitter (Dopamin, Serotonin, Adrenalin, Noradrenalin) sind und zum anderen fungieren sie im zentralen Nervensystem selbst als Neurotransmitter (Glutaminsäure und Glycin).

Insofern sind die Aminosäuren schon für gesunde Menschen von herausragender Bedeutung, insbesondere wenn es um Leistungen des Gehirns geht, (vor allem beim Lernen und Gedächtnisübungen) die das Lernen betreffen sowie das Arbeitsgedächtnis und die Konzentration.

Die in der Tabelle 1 dargestellten Werte orientieren sich besonders am Bedarf von Kindern und Jugendlichen und sind somit genau auf diese Altersgruppe abgestimmt.

Ein weiterer Anspruch dieses Getränks ist, die gezielte Unterstützung und Versorgung von Kindern und Jugendlichen mit dem ADHS (Aufmerksamkeitsdefizit-Hyperaktivitäts-Syndrom) sowie mit ADS (AufmerksamkeitsdefizitSyndrom). Dabei betrifft ADHS und ADS nach Kriterien des DSM-IV 4 bis 8 Prozent aller Schulkinder in Deutschland. Die Prävalenz von ADHS/ADS im Erwachsenenalter wird mit 1,3 bis 4,7 Prozent angegeben.

Besonders bevorzugt findet das erfindungsgemäße Getränk daher Verwendung in der Prophylaxe und/oder Behandlung von Aufmerksamkeitsdefizithyperaktivitätssyndrom (ADHS) und/oder Aufmerksamkeitsdefizitsyndrom (ADS).

Ganz besonders eignet sich das erfindungsgemäße Getränk zur Verwendung in der Prophylaxe und/oder Behandlung von ADHS und/oder ADS bei Kindern und Jugendlichen.

Hierbei wurde gefunden, dass die in Tabelle I genannte Zusammensetzung besonders gut für die Behandlung von ADHS und/oder ADS geeignet ist.

Dopamin ist ein Zwischenprodukt in der Biosynthese von Adrenalin ausgehend von der Aminosäure Tyrosin. Tyrosin wird durch Tyrosinhydroxylase in Levodopa umgewandelt und dieses wiederum durch Aromatische-L-Aminosäure-Decarboxylase in Dopamin.

Für den menschlichen Organismus sind die Aminosäuren Lysin, Methionin, Threonin, Isoleucin (Aspartatfamilie), Valin und Leucin (Pyrovatfamilie), Phenylalanin und Tryptophan (Shikimisäurefamilie) essentiell.

Semi-essentielle Aminosäuren müssen nur in bestimmten Situationen mit der Nahrung aufgenommen werden, zum Beispiel während des Wachstums oder bei schweren Verletzungen. Die übrigen Aminosäuren werden entweder direkt synthetisiert oder aus anderen Aminosäuren durch Modifikation gewonnen. Cystein kann aus der essentiellen Aminosäure Methionin synthetisiert werden.

Für Kinder ist zusätzlich zu den generell essentiellen Aminosäuren Tyrosin essentiell, da in diesem Lebensalter die Körperfunktion zu dessen Herstellung aus Phenylalanin noch nicht ausgereift ist (Löffler et al., Lehrbuch der Biochemie und Pathobiochemie, Springer Verlag, 2006).

In dem erfindungsgemäßen Getränk ist Phenylalanin enthalten, da Phenylalanin die Ausgangssubstanz für die Tyrosinbildung darstellt. Tyrosin wiederum ist das Vorläufermolekül für die Synthese der Katecholamine Dopamin, Adrenalin und Noradrenalin. In einer kanadischen Studie von 1991 wurden erniedrigte Konzentrationen von Phenylalanin und Tyrosin bei ADHS festgestellt.

Dabei muss aber erwähnt werden, dass zum Thema ADHS und Aminosäurenkonzentrationen bisher nur wenige Studien vorliegen. In der orthomolekularen Medizin wird eine Phenylalanin- und/oder Tyrosinsupplementierung häufig mit gutem Erfolg bei verringerter kognitiver Leistungsfähigkeit, verminderter Stresstoleranz und bei depressiven Verstimmungen eingesetzt (Eisenberg et al., J. Clin. Psychiatry (1988) 49:195).

Des Weiteren ist in dem erfindungsgemäßen Getränk Tryptophan enthalten, da Tryptophan die Ausgangssubstanz für die Bildung des Neurotransmitters Serotonin und des Epiphysenhormons Melatonin ist. Serotonin spielt eine wichtige Rolle für die Regulierung von Stimmung und Sozialverhalten. Die Serotoninverfügbarkeit im Gehirn hängt in hohem Maße von der Tryptophankonzentration im Blutserum ab.

In zahlreichen Studien, in denen Versuchspersonen ein tryptophanfreies Eiweißgetränk verabreicht wurde, konnten anschließend Veränderungen der Stimmungslage, des Sozialverhaltens etc. festgestellt werden. Aus diesem Grund kann eine Nahrungsergänzung mit Tryptophan auch bei Kindern mit Hyperaktivität hilfreich sein, sofern niedrige Tryptophan- oder Serotoninkonzentrationen gemessen wurden (Zepf et al., Hum. Psychopharmacol. (2007), Oct. 9).

Diese Versuche deuten ferner generell darauf hin, dass das erfindungsgemäße Getränk für die Prophylase und/oder Behandlung von psychischen Störungen im Allgemeinen wirksam ist. Bevorzug wird das erfindungsgemäße Getränk deshalb als Medikament verwendet, bevorzugt als Medikament in der Behandlung von psychischen Störungen.

Die Blut-Hirnschranke stellt eine wichtige Barriere zwischen Blutserum und Liquor dar. Dabei ist die Konzentrationen der Aminosäuren im Blutserum und ihr Verhältnis zueinander von entscheidender Bedeutung, wie hoch der Anteil der die Blut-Hirn-Schranke passierenden Aminosäuren ist. So nimmt beispielsweise die Serotoninkonzentration im Gehirn schon nach einer tryptophanfreien Mahlzeit messbar ab.

Eine japanische Studie zeigte hierfür einen Zusammenhang zwischen ADHS bei Kindern und einer Störung des Tryptophan-Serotonin-Metabolismus (Hoshino et al., Folia Psychiatry Neurol. Jpn. (1985) 39:531).

Außerdem fand eine Studie der Ohio State University bei ADHS-Kindern niedrigere Plasmakonzentrationen von Phenylalanin, Tyrosin, Tryptophan, Histidin und Isoleucin als bei Kontrollpersonen (Bornstein et al., Psychiatry Res. (1990) 33:301). Ferner fand eine kanadische Studie ebenfalls erniedrigte Konzentrationen von Phenylalanin und Tyrosin bei ADHS-Patienten (Baker et al., Biol. Psychiatry (1991) 29:15).

Überdies stellte eine Arbeitsgruppe aus Venezuela fest, dass bei ADHS-Patienten und Patienten mit autistischen Störungen im Vergleich zu einer Kontrollgruppe niedrigere Phenylalanin- und Glutamin- Serumkonzentrationen haben - bei gleichzeitig erhöhten Glycin-Konzentrationen (Zavala et al., Rev. Neurobiol. (2001) 33:401).

In einer anderen Studie, in welche die Supplementierung von S-AdenosylMethionin (SAM) bei erwachsenen ADHS-Patienten gemessen wurde, konnte bei 75 % der Studienteilnehmer eine Besserung der ADHS-Symptomatik durch die Supplementierung festgestellt werden. SAM wird für die Synthese von Serotonin und Adrenalin benötigt (Shekim et al., Psychopharmacol. Bull. (1990) 26:249).

Hinzukommend konnte in einer randomisierten Doppelblindstudie gezeigt werden, dass eine Carnitin-Supplementierung zu einer signifikanten Verbesserung der ADHS-Symptomatik bei Schülern führt (Van Oudheusden & Scholte, Prostaglandins (2002) 67:33).

Somit zeigt sich anhand der verschiedenen Studien, dass auch bei Kindern und Jugendlichen mit einem ADHS-Syndrom das hier vorgestellte Getränk zur Unterstützung herangezogen werden kann.

Anhand des nachfolgenden Beispiels soll der erfindungsgemäße Gegenstand näher erläutert werden, ohne diesen auf die hier gezeigte spezifische Ausführungsform einschränken zu wollen.

### Beispiel - Erfindungsgemäßes Getränk auf Holundersaftbasis

Als Beispiel wird hier in Tabelle II ein Getränk auf Basis des Holunderbeerensaftes ausgearbeitet. Dieses Beispiel wurde deshalb gewählt, da aufgrund der Fruchtbasis schon sehr viele Inhaltsstoffe vorhanden sind und infolgedessen nur noch wenige hinzugefügt werden müssen. Die weiteren Werte basieren **auf den oben genannten Mittelwerten.**

| **Tabelle II** | |
|---|---|
| **Flüssigkeit** | **Menge** |
| Mineralwasser | 96,28357 ml |
| Fruchtsaft der Holunderbeere | 100 ml |

| **Aminosäuren** | **Menge** |
|---|---|
| Phenylalanin | 660 mg |
| Tyrosin | 435 mg |
| Tryptophan | 144mg |
| Arginin | 750 mg |
| Histidin | 360 mg |
| *Summe (Aminosäuren):* | Summe: 2349 mg (ca. 2,349 ml) |

| **Vitamine** | **Menge** |
|---|---|
| Vitamin C | 82,5 mg |
| Vitamin D | 0,0045 mg |
| Vitamin B1 | 1,05 mg |
| Vitamin B2 | 1,15 mg |
| Vitamin B6 | 0,75 mg |
| Vitamin B7 | 0,019 mg |
| Vitamin B12 | 1,75 mg |
| *Summe (Vitamine):* | 5,430 mg (ca. 0,00543 ml) |

| **Mineralstoffe** | **Menge** |
|---|---|
| Eisen | 11 mg |
| Zink | 6,5 mg |
| Calcium | 950 mg |
| Magnesium | 180 mg |
| *Summe (Mineralstoffe):* | 1362 mg (ca. 1,362 ml) |
| **Gesamtsumme:** | **200 ml** = **0,2 l** |

## Patentansprüche

1. Aminosäurehaltiges Getränk enthaltend auf 200 Gramm Wasser
- 440-880 mg Phenylalanin,
- 290-580 mg Tyrosin sowie
- 96-192 mg Tryptophan,
mit der Maßgabe, dass die Menge von Phenylalanin größer als die Menge von Tyrosin und Tryptophan und die Menge von Tyrosin größer ist als die Menge an Tryptophan ist,
sowie als Vitamine
- 0,5-1,0 mg Vitamin B6.

2. Getränk nach Anspruch 1 **dadurch gekennzeichnet, dass** es zusätzlich als Mineralstoffe
- 5 mg - 8 mg Zink und
- 120 mg - 240 mg Magnesium
enthält.

3. Getränk nach Anspruch 1 oder 2 **dadurch gekennzeichnet, dass** es zusätzlich als Aminosäuren Arginin und/oder Histidin enthält.

4. Getränk nach Anspruch 1 bis 3 **dadurch gekennzeichnet, dass** es zusätzlich als Vitamine, Vitamin C, Vitamin D, Vitamin B1, Vitamin B2, Vitamin B7 und/oder Vitamin B12 enthält.

5. Getränk nach mindestens einem der Ansprüche 1 bis 4, **dadurch gekennzeichnet, dass** es als zusätzliche Mineralstoffe Eisen und/oder Calcium enthält.

6. Getränk nach mindestens einem der Ansprüche 1 bis 5 **dadurch gekennzeichnet, dass** es
- 440-880 mg Phenylalanin,
- 290-580 mg Tyrosin,
- 96-192 mg Tryptophan,
- 500-1000 mg Arginin,
- 240-480 mg Histidin sowie
- 75-90 mg Vitamin C,
- 0,04-0,005 mg Vitamin D,
- 1,0-1,1 mg Vitamin B1,
- 1,0-1,3 mg Vitamin B2,
- 0,5-1,0 Vitamin B6,
- 0,013-0,025 mg Vitamin B7 sowie
- 1,5-2.0 mg Vitamin B12 und
- 8,0-14 mg Eisen,
- 5,0-8,0 mg Zink,
- 700-1100 mg Calcium und
- 120-240 mg Magnesium
enthält.

7. Getränk nach mindestens einem der Ansprüche 1 bis 6, **dadurch gekennzeichnet, dass** das Getränk aus Wasser und zugesetzten Fruchtsäften sowie zugesetzten Aminosäuren, Vitaminen und/oder Mineralstoffen gebildet worden ist.

8. Getränk nach Anspruch 7 **dadurch gekennzeichnet, dass** das Getränk aus Mineralwasser und Holundersaft sowie zugesetzten Aminosäuren, Vitaminen und Mineralstoffen gebildet worden ist.

9. Getränk nach mindestens einem der Ansprüche 1 bis 8 **dadurch gekennzeichnet, dass** es kein Koffein enthält.

10. Getränk nach mindestens einem der Ansprüche 1 bis 9 **dadurch gekennzeichnet, dass** es kein Taurin enthält.

11. Getränk nach mindestens einem der Ansprüche 1 bis 10, **dadurch gekennzeichnet, dass** es Kräuter-, Tee-, Gemüse-, Obst- und/oder Gewürzauszüge enthält.

12. Getränk nach einem der Ansprüche 1 bis 11 zur Verwendung als Medikament, bevorzugt als Medikament zur Behandlung von psychischen Störungen.

13. Getränk nach einem der Ansprüche 1 bis 12 zur Verwendung in der Prophylase und/oder Behandlung von Aufmerksamkeitsdefizithyperaktivitätssyndrom (ADHS) und/oder Aufmerksamkeitsdefizitsyndrom (ADS).

14. Getränk nach einem der Ansprüche 1 bis 13 zur Verwendung in der Prophylaxe und/oder Behandlung von ADHS und/oder ADS bei Kindern und/oder Jugendlichen.

15. Verwendung eines Getränks nach mindestens einem der Ansprüche 1 bis 11 zur Prophylaxe und/oder Therapie von Kindern und/oder Jugendlichen mit ADHS und/oder ADS.
